Europäisches Patentamt

European Patent Office

Office européen des brevets

(19)

(11) Publication number: **0 372 524**
**A2**

# (12) EUROPEAN PATENT APPLICATION

(21) Application number: **89122482.6**

(22) Date of filing: **06.12.89**

(51) Int. Cl.⁵: **C12N 15/10, C12P 19/34,**
**//C12Q1/68**

(30) Priority: **07.12.88 US 280866**

(43) Date of publication of application:
**13.06.90 Bulletin 90/24**

(84) Designated Contracting States:
**AT BE CH DE ES FR GB GR IT LI LU NL SE**

(71) Applicant: **THE GENERAL HOSPITAL**
**CORPORATION**
**55 Fruit Street**
**Boston MA 02114(US)**

(72) Inventor: **Straus, Donald**
**28 Shepard Street**
**Cambridge Massachusetts 02138(US)**
Inventor: **Ausubel, Frederick, Dr.**
**271 Lake Avenue**
**Newton MA 02161(US)**

(74) Representative: **Klein, Otto, Dr. et al**
**Hoechst AG Zentrale Patentabteilung**
**Postfach 80 03 20**
**D-6230 Frankfurt am Main 80(DE)**

(54) **Method of enrichment and cloning for DNA containing an insertion or corresponding to a deletion.**

(57) The present invention concerns a method for enriching for, and optionally, clonally isolating, gene sequences which contain an insertion or which correspond to deletion. The method is capable of such enrichment and isolation even when the insertion or deletion has not been genetically characterized, or is incapable of causing a discernible deficiency.

GENOMIC SUBTRACTION

FIG. 1

# METHOD OF ENRICHMENT AND CLONING FOR DNA CONTAINING AN INSERTION OR CORRESPONDING TO A DELETION

## Field of the Invention

The invention pertains to recombinant DNA technology, and, more specifically, to a method for enrichment and cloning of a desired nucleic acid molecule.

## Background of the Invention

The ability to identify and clone a particular, desired gene sequence from a virus, prokaryote or eukaryote is of tremendous significance to molecular biology and medicine. Such cloned gene sequences can be used to diagnose the presence of a disease or the predilection of a patient to manifest a disease. Such cloned gene sequences can also be used to express a desired gene product and therefore can potentially be used for applications ranging from catalysis to gene replacement. The study of such expressed products can provide insight into the pathology and treatment of human disease.

A variety of methods have been developed for isolating and cloning desired gene sequences. Early methods permitted only the identification and isolation of gene sequences which possessed a unique property such as proximity to a prophage integration site, capacity for self-replication, distinctive molecular weight, extreme abundance, etc. (The Bacteriophage Lambda, A.D. Hershey, d., Cold Spring Harbor Press, Cold Spring Harbor, NY (1971); Miller, J.H. Experiments in Molecular Genetics, Cold Spring Harbor Press, Cold Spring Harbor, NY (1972); Molecular Biology of the Gene, Watson, J.D. et al., (4th ed.) Benjamin/Cummings, Menlo Park, CA (1987); Darnell, J. et al. Molecular Biology, Scientific American Books, NY, NY (1986)). Because these methods relied upon distinctive properties of a gene sequence, they were largely (or completely) unsuitable for identifying and cloning most gene sequences.

In order to identify desired gene sequences which lacked a distinctive property, well characterized genetic systems (such as Escherichia coli, Saccharomyces cerevisiae, maize, mammalian cells, etc.) have been exploited. In accordance with this method, cells are mutagenized (by chemical (such as UV light, hydroxylamine, etc. (Miller, J.H. Experiments in Molecular Genetics, Cold Spring Harbor Press, Cold Spring Harbor, NY (1972)) or genetic means (such as transposon tagging (Davis, R.W. et al. A Manual for Genetic Engineering, Advanced Bacterial Genetics, Cold Spring Harbor Press, Cold Spring Harbor, NY (1980)) to produce mutants having discernible genetic deficiencies. A desired gene sequence is then identified by its capacity to complement (i.e. remedy) the genetic deficiencies of such mutant cells. Such genetic identification permitted the genetic characterization of the gene sequences, and the construction of genetic maps which localized the gene sequence to a region of a particular chromosome (Taylor, Bacteriol. Rev. 34:155 (1970)).

With the advent of recombinant DNA technologies, it became possible to clone (i.e. to physically isolate) such genetically characterized gene sequences. Random fragments of a genome could be introduced into self-replicating vectors to produce gene libraries, each of whose members contain a unique DNA fragment (Maniatis, T. et al., In: Molecular Cloning, a Laboratory Manual, Cold Spring Harbor Press, Cold Spring Harbor, NY (1982)). By screening the members of such libraries for those capable of complementing the deficiency of a mutant cell, it was possible to clone the desired gene sequence.

Although these methods permit the identification and cloning of many gene sequences, they may be employed only where a host cell exists which has a mutation conferring a discernible deficiency, and the gene sequence can be cloned into a gene sequence delivery system (such as a vector) capable of entering the host cell and being expressed.

The capacity to physically isolate certain gene sequences has led to the development of methods which are capable of isolating a desired gene sequence even in the absence of mutations or vectors.

In one such technique, known as "chromosome walking," a desired sequence can be obtained by isolating a gene sequence which is capable of hybridizing to a particular reference sequence. This isolated gene sequence is then employed as a reference sequence in a subsequent hybridization experiment in order to clone a gene sequence which is adjacent to, and which partially overlaps, the originally isolated sequence. This newly isolated sequence will be physically closer to the desired gene sequence that was the originally isolated sequence. This process is repeated until the desired gene sequence has been obtained. As will be appreciated, the ability to clone a gene sequence, in the absence of genetic mutants or vectors, requires some initial information concerning the nucleotide sequence or restriction endonuclease digestion profile of the desired sequence.

Alternatively, the chromosome of a virus or cell can be characterized to produce a physical map based on either nucleotide sequence or restriction endonuclease cleavage data (i.e. an RFLP map). Using such a map, restriction fragments of the chromosome can be cloned without any prior determination as to their genetic function.

More recently, gene cloning has been achieved by producing synthesizing oligonucleotide molecules whose sequence has been deduced from the amino acid sequence of an isolated protein, by forming cDNA copies of isolated RNA transcripts, by differential colony or library subtractive hybridizations using either two different RNA sources, or cDNA and RNA.

Although these methods may be employed even in the absence of either mutants or a gene sequence delivery system, they permit a desired gene sequence to be identified and cloned only if sequences naturally linked to the desired sequence have been characterized and isolated, or if the sequence or restriction map of such sequences has been obtained. Since such data is often unavailable, these methods are often incapable of use in identifying and cloning a desired gene sequence.

Thus, in summary, the ability to clone DNA corresponding to a locus defined only by a mutation is a relatively simply matter when working with E. coli, S. cerevisiae or other organisms in which transformation and complementation with genomic libraries is feasible. Chromosome walking techniques may be used in other organisms to clone genetically defined loci if the mutant was obtained by transposon tagging, if the locus can be linked to markers in an RFLP map, or if an ordered library for the genome exists. Unfortunately, there are numerous organisms in which mutants with interesting phenotypes have been isolated but for which such procedures have not been developed. Thus, many gene sequences cannot be isolated using the above methods.

Existing techniques can, in general, only be applied to permit the identification and isolation of a desired gene sequence if certain additional conditions are fulfilled. These conditions require the existence of either genetic or structural data. In view of these constraints, methods capable of permitting, or facilitating, the identification and cloning of gene sequences which cannot easily be isolated using current techniques are of great interest.

## Brief Description of the Figure

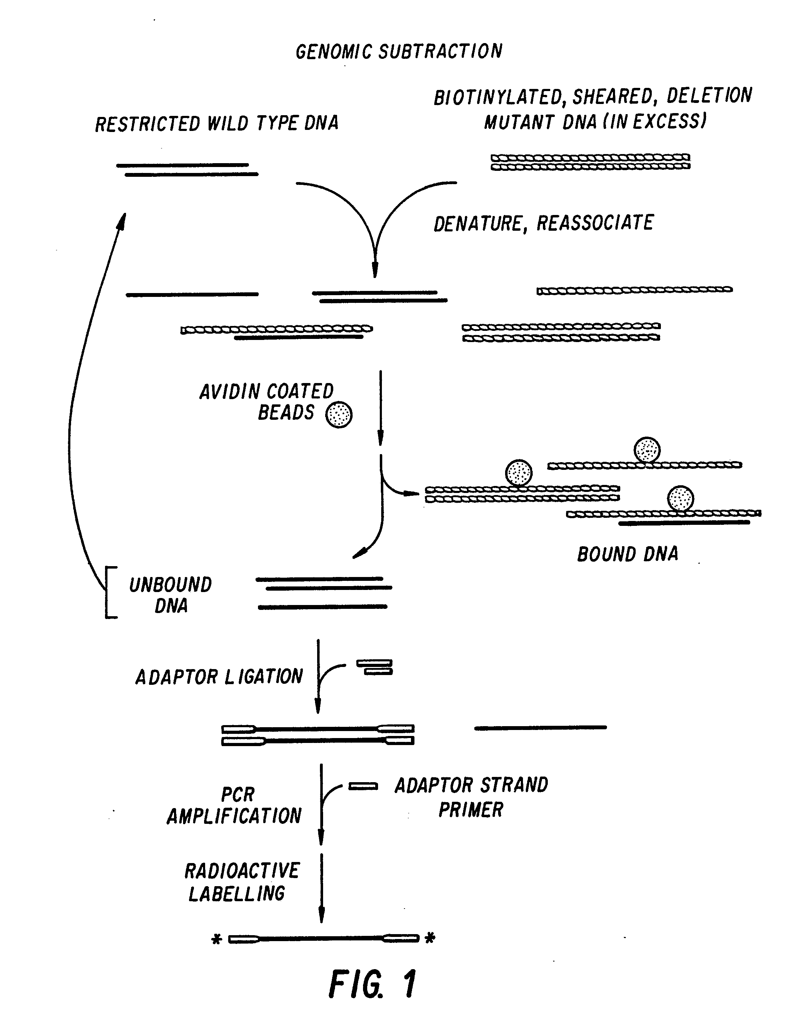

Figure 1 is a diagramm of the enrichment and cloning method of the preferred embodiment of the present invention. DNA is depicted as a solid line; biotinylated DNA is depicted as a striped black/white line; Sau3a adaptors are shown as an open line; avidin beads are shown as speckled circles; radiolabelled fragments are shown with asterisks.

## Summary of the Invention

A technique, called "genomic subtraction," has been developed for isolating the DNA that is absent in deletion mutants. The method removes from wild-type DNA all of the sequences that are present in both the wild-type and the deletion mutants genomes. The DNA that remains is derived from the deleted region. The method of genomic subtraction has been exemplified by cloning DNA from a yeast strain corresponding to a 5 kb yeast DNA deletion. In brief, the method achieves an enrichment for the deleted sequences by allowing wild-type DNA to reassociate with an excess of biotinylated deletion mutant DNA. After reassociation, the biotinylated sequences are removed by binding to avidin coated beads. This subtraction process was then repeated several times. In each cycle the unbound wild-type DNA from the previous round was hybridized with fresh biotinylated deletion mutant DNA. Samples of unbound DNA from each cycle were capped with adaptors (such as Sau3A adapters) and amplified by using one strand of the adaptor as a primer in the polymerase chain reaction. Replicas of a wild-type yeast genomic library were probed with the labelled amplified products. All of the genomic clones that hybridized to the labelled DNA from the third round of subtraction were found to contain sequences that are deleted in the mutant.

The present invention thus concerns a method for enriching for, and optionally clonally isolating, gene sequences which contain an insertion or which correspond to deletion. Of special concern is a method capable of such enrichment and isolation even when the insertion or deletion has not been genetically characterized, or is incapable of causing a discernible deficiency.

In detail, the invention provides a method for enriching for a desired gene sequence absent in one population of nucleic acid molecules but present in another which comprises:

a) mixing a first population of denatured nucleic acid molecules suspected of possessing the desired gene sequence with an excess amount of a second population of denatured nucleic acid molecules suspected of lacking the desired gene sequence, wherein the second population of molecules has been labelled with a label, and wherein the first population of molecules lacks the label;

b) permitting the mixed molecules of the first and second populations of molecules to hybridize and thereby form double-stranded nucleic acid molecules; and

c) isolating any double-stranded molecules of the mixture which are composed solely of renatured molecules of the first population.

The invention further concerns the above-described methods wherein step (c) comprises the sub-steps:

1. incubating the mixed first and second populations of molecules with an agent capable of binding to the label of the second population of molecules;

2. permitting the agent to bind to the label of the second population of molecules; and

3. separating any of the second population of molecules from the mixture by selectively separating the bound agent, along with its bound, labelled, molecule of the second population of molecules, from the mixture.

The invention further concerns the above-described methods wherein the label of the labelled second population of molecules is a biotin label, or an antibody label.

The invention further concerns the above-described methods wherein the agent is an antibody or avidin, either in solution, or bound (as to a bead or resin).

The invention also concerns the above-described methods wherein the avidin in solution, after binding to the labelled molecule of the second population of molecules, is removed from solution along with the bound, labelled, molecule by affinity separation with biotin, or by immunoaffinity separation with an anti-avidin antibody.

## Description of the Preferred Embodiments

The present invention concerns a method for enriching for, and clonally isolating a gene sequence present in one nucleic acid population but absent in another.

As used herein, the term "gene sequence" is intended to refer to a nucleic acid molecule which may be either DNA or RNA. The preferred Nucleic acid molecule composing the gene sequence will be a DNA molecule. The gene sequence may be of any length, and may possess any nucleotide sequence. The gene sequence may be capable of expressing a gene product (i.e. it may possess sufficient regulatory sequences to enable its transcription into RNA and its translation into a protein or polypeptide), or, alternatively, it may be incapable of such expression. The term "gene sequence" is further intended to include sequences which are non-transcribed (such as regulatory sequences), non-translated (such as introns, and processing sequences), or which encode genes or gene fragments. A "gene sequence" may contain more than one such sequence. The gene sequences of the present invention may be viral, prokaryotic or eukaryotic, or may be synthetic or recombinant.

The desired gene sequences of concern to the present invention are "present in one nucleic acid population but absent in another."

Such a desired gene sequence may comprise an insertion which is present in one sequence but absent in another. Examples of such an insertion include an integrated virus, gene duplication, mutational insertion, transposon insertion, etc. Alternatively, such a desired gene sequence may comprise an extrachromosomal element such as a plasmid, episome, virus, etc. which is present in one source but absent from another. Such a desired gene sequence may alternatively comprise a sequence which is incapable of hybridizing with a second sequence (such as by being highly divergent with respect to the second sequence). The desired gene sequence may alternatively comprise a sequence which corresponds to a deletion that is present in a particular reference sequence. The term "deletion" is intended to refer to the absence of a particular sequence of nucleotides from a particular DNA or RNA molecule which normally and naturally contains the sequence.

A nucleic acid sequence is said to "have a deletion" if, when compared to a second nucleic acid sequence, it is found to lack at least one nucleotide which is present in that sequence. For example, if one nucleic acid sequence contains the sequence "ATGCGTAA," and a second sequence contains the sequence "ATGAA," then the second sequence is said to have a deletion of the sequence "CGT." The deleted nucleotides ("CGT") being between "ATG" and "AA."

A nucleic acid sequence is said "to have or to carry an insertion" or "to correspond to the deletion" if it contains a sequence which corresponds to all or some of the sequence of nucleotides which is absent from the sequence which has the deletion. Thus, referring to the above example, the sequence "CGT" corresponds to the sequence deleted in the sequence "ATGAA."

The expressions "contain an insertion" or "correspond to a deletion" are intended to be equivalent, and to reflect merely whether ones bases ones reference point on the molecule containing the deletion or on the molecule which corresponds to the deletion. Thus, for example, if a particular sequence, normally present in a nucleic acid molecule, is absent from the nucleic acid molecule of a mutant, then the mutant is said to "have or carry" a deletion of these sequences. The present invention permits one to enrich for and clonally isolate a nucleic acid molecule which corresponds to this deletion.

Similarly, if a particular sequence is normally not present in a nucleic acid molecule (such as the sequence of an integrated virus), but is present in the nucleic acid molecule of a mutant, then the mutant is said to "have or carry" an insertion of these sequences (i.e. to have or carry a sequence which corresponds to a "deletion" in the non-mutant). The present invention permits one to enrich for and clonally isolate a nucleic acid molecule which contains this insertion (i.e. which corresponds to this "deletion").

The methods of the present invention are equally applicable regardless of whether the deletion results from the loss of a nucleotide sequence from a reference nucleic acid molecule or whether it reflects an insertion in one nucleic acid molecule which is not present in a reference nucleic acid molecule. Importantly, therefore, the term "deletion" denotes only that a comparison has been made between two nucleic acid sequences, and that one sequence has been found to lack a sequence found in the other. As used herein, the sequence which is "lacked" by one molecule is known as the deletion. The sequence in the other molecule which corresponds to the deletion may be equivalently either an insertion or a sequence which "corresponds to the deletion."

One object of the present invention is to achieve the "enrichment" of gene sequences which correspond to a particular deletion (or which contain an insertion). As used herein, the term "enrichment" is intended to refer to an increase in the concentration of a particular gene sequence with respect to the total concentration of gene sequences in a sample.

Once a desired gene sequence has been enriched for, it is possible to insert the enriched gene sequence into a suitable viral or plasmid vector and thereby obtain the clonal isolation of the enriched gene sequence. Alternatively, the sequence can be labeled and used to probe other sequences. Methods which can be used to accomplish such insertion are disclosed, for example, by Maniatis, T. et al. (In: Molecular Cloning, a Laboratory Manual, Cold Spring Harbor Press, Cold Spring Harbor, NY (1982)).

I. Methods of Deletion/Insertion Cloning

Two general approaches have been described for cloning sequences that are present in one strain and absent in another. The first approach, differential screening, has been used to clone the esc gene from Drosophila. Using genomic DNA from strains with and without a deletion to probe replicas of a genomic library poses technical difficulties which become daunting for large genomes. In addition, the deletion must cover at least one entire insert in the genomic library which does not contain any repeated sequences.

The second approach, competitive hybridization, provides an elegant alternative to differential screening. This technique was used by Lamar et al. (Cell 37:171-177 (1984)) to isolate clones specific for the human Y chromosome. In accordance with this method, an excess of sheared DNA from a human female is denatured and reannealed along with a small amount of DNA from a male (the male-derived DNA having been previously treated to have sticky ends). Most of the male DNA reassociated with the sheared DNA yielding unclonable fragments lacking sticky ends. Fragments unique to the Y chromosome, however, could only reassociate with the complementary restricted strand (derived from the Y chromosome). Such reassociation thus formed clonable fragments with sticky ends. This technique has also been used successfully to clone DNA corresponding to deletions in the Duchenne muscular dystrophy locus, and choroideremia.

Unfortunately, the competitive hybridization method does not provide a large enough degree of enrichment. For example, enrichments of about one hundred fold were obtained for the sequences of interest in the above experiments. With enrichments of such low magnitude, the technique is practical only when dealing with large deletions. Indeed, even if the deletion covered 0.1% of the genome, many putative positive clones have to be tested individually by labeling and probing genomic Southern blots (Southern, J., J. Molec. Biol. 98:503-517 (1975)). The method as it stands, then, is not practical for deletions on the order of 1 kbp (kilobasepair) unless one is dealing with a small prokaryotic genome.

II. THE DELETION/INSERTION CLONING METHOD OF THE PRESENT INVENTION

The present invention achieves the enrichment of gene sequences which correspond to a deletion using iterative rounds of subtractive hybridization to enrich for sequences absent in one population of nucleic acid molecules but present in another. The method is diagrammed in Figure 1.

In accordance with the methods of the present invention, two sources of nucleic acid are required. The first source contains a nucleic acid molecule which lacks a sequence present in the nucleic acid of a second source. The sequence which is absent from the first source (the source which "contains a deletion") but which is present in the second source (the source which "contains a sequence which corresponds to the deletion") is the "desired sequence" whose enrichment and cloning is the object of the methods of the present invention.

The deletion carried in the nucleic acid of the first source can be induced artificially, as through the use of mutagens or transposable elements (Miller, J.H. Experiments in Molecular Genetics, Cold Spring Harbor Press, Cold Spring Harbor, NY (1972); Davis, R.W. et al. A Manual for Genetic Engineering Advanced Bacterial Genetics, Cold Spring Harbor Press, Cold Spring Harbor, NY (1980)), etc., alternatively, the deletion may arise naturally during evolution, and thus reflect the genetic relatedness (or lack thereof) between the two sources.

The nucleic acid of the two sources is extracted and mixtures of nucleic acid molecules are purified from other cellular components. For this purpose, one may follow the procedures of Maniatis, T. et al., In: Molecular Cloning, a Laboratory Manual, Cold Spring Harbor Press, Cold Spring Harbor, NY (1982)).

The nucleic acid molecule which carries the deletion may be either DNA or RNA. The nucleic acid molecule which corresponds to the deletion may also be either DNA or RNA. However, due to the desirability of amplifying this molecule after its enrichment, it is preferable for the nucleic acid molecule which corresponds to the deletion to be a DNA molecule. The most preferred embodiment of the present invention employs DNA sources for both the deletion gene sequence and the desired gene sequence. In the following discussion of the invention, the invention will be described in terms of this preferred embodiment.

Once a nucleic acid sample has been obtained which contains the deletion gene sequence, this sample is treated to decrease the size of the nucleic acid molecules which it contains. For this purpose, one or more restriction endonucleases (if the sample is DNA) are most preferably employed. The most preferred restriction endonuclease is Sau3a, although other restriction endonucleases can be employed. It is desirable for the nucleic acid molecule to be cleaved into fragments which range from 0.1 -100 kb in size, and more preferably, 0.1 - 10 kb in size. For a deletion of approximately 5 kb, the DNA is preferably reduced to approximately 3 kb in size. Methods for cleaving DNA molecules with restriction endonucleases are provided by Maniatis, T. et al., In: Molecular Cloning, a Laboratory Manual, Cold Spring Harbor Press, Cold Spring Harbor, NY (1982)).

The sample which contains the nucleic acid molecule that carries the deletion is sheared, using conventional means, such that the average length of the nucleic acid molecules is similar to that of the above-described molecules. The nucleic acid of this sample is modified, preferably with biotin, such that the molecules will become able to bind to free or immobilizable avidin (such as an avidin bead or column (U.S. Patent No. 4,228,237 (Hevey et al.); Hofman et al., J. Amer. Chem. Soc. 100:3585-3590 (1978)). The modification is most preferably accomplished using photobiotin (Vector Laboratories). Other methods can, of course, be employed to produce such biotinylated molecules.

Once the above procedures have been accomplished, the two treated nucleic acid samples are mixed and incubated under conditions sufficient to cause denaturation of any double stranded molecules. It is, alternatively, possible to denature the separated preparations, and then to mix the single-stranded preparations together. The nucleic acid samples are to be mixed together such that the concentration of the nucleic acid molecule which carries the deletion will be in excess over the concentration of the nucleic acid molecule which corresponds to the deletion.

Once mixing/denaturation has been accomplished, the mixed sample is cooled and incubated under conditions sufficient to permit hybridization and the reassociation of complementary strands. The conditions of such hybridization can be controlled to enhance or optimize the rate of hybridization. For example, high concentrations of salts such as 1 M NaCl, Dextran Sulfate, 4 M Lithium Chloride, 2 M Ammonium Sulfate, etc. may be used. Such incubation will result in the production of five classes of nucleic acid molecules:

(1) Double-stranded molecules both of whose strands are biotinylated. Such molecules result from the reannealing of two strands both derived from the sample containing the nucleic acid molecule which carries the deletion. Since both stands are biotinylated, and thus derived from the source which carried the deletion, no member of this class of molecules can contain the desired gene sequence (which corresponds to the deletion and therefore must come from the other source).

(2) Double-stranded molecules having only one biotinylated strand. Such molecules result from the reannealing of a strand derived from the sample containing the nucleic acid molecule which corresponds to the deletion, and a strand derived from nucleic acid molecule of the sample which contained the deletion. Since such molecules are hybrids of both sources of nucleic acid, all members of this class of molecules contain sequences which are unrelated to the deletion sequence (since that sequence is not present in the biotinylated nucleic acid source).

(3) Single-stranded biotinylated molecules. Such molecules result from the failure of a nucleic acid molecule derived from the deletion containing source to reanneal to another strand. Since this class of molecules is derived from the sample containing the nucleic acid molecule which carries the deletion, no member of this class of molecules can contain the desired gene sequence.

(4) Single-stranded non-biotinylated molecules. Such molecules result from the failure of a nucleic acid molecule derived from the source containing a nucleic acid molecule which corresponds to the deletion to reanneal to another strand. Since this class of molecules is derived from the sample containing the desired gene sequence, members of this class of molecules potentially contain the desired gene sequence.

(5) Double-stranded molecules neither of whose strands are biotinylated. Such molecules result from the reannealing of two strands both derived from the sample containing the nucleic acid molecule which corresponds to the deletion. Since this class of molecules is derived from the sample containing the desired gene sequence, members of this class of molecules potentially contain the desired gene sequence. Members of this class of molecules are especially desirable since their double-stranded nature permits them to be incorporated into cloning vectors.

As will readily be perceived in light of the above discussion, the number of molecules which fall into classes 4 and 5 will decrease as more of the biotinylated, deletion-containing nucleic acid is added to the mixture. Thus by having such biotinylated DNA in excess, the amount of class 4 or class 5 molecules (relative to the amounts of class 1, 2, and 3 molecules) will be low.

Despite the fact that the unwanted molecules of classes 1, 2, and 3 will be present in great excess over the desired molecules of classes 4 and 5, it is possible to easily remove the unwanted molecules from the mixture by virtue of the fact that the molecules of classes 1, 2 and 3 are biotinylated.

Such biotinylated molecules, whether hybridized to a complementary biotinylated molecule (class 1), hybridized to a complementary non-biotinylated molecule (class 2), or single-stranded (class 3), can be removed from the sample in any of a variety of ways. Such molecules can be incubated in the presence of free avidin in solution or suspension, under conditions sufficient to permit the avidin to bind to the biotin of the biotinylated molecules. The preparation may then be incubated in the presence of bound biotin (for example, biotinylated beads, resins, columns, etc.) under conditions sufficient to permit the biotinylated nucleic acid bound-avidin to bind to the biotin of the column (avidin possesses multiple biotin binding sites, and can, thus, simultaneously bind to the biotinylated nucleic acid molecule and to the biotinylated bead or resin). An immobilized avidin column is most preferably used for this purpose. In lieu of an avidin column, it is possible to employ other methods for removing the biotinylated molecules from the mixture. For example, one could employ a streptavidin-cellulose matrix, avidin-agarose or avidin coupled to magnetic beads, etc. Unbound molecules of classes 4 and 5 can then be recovered from the eluate of the biotinylated column, or from the supernatant of the centrifuged biotinylated bead, etc.

In an alternative, preferred, method, the biotinylated molecules of classes 1-3 are incubated in the presence of avidin beads, resins, etc. (i.e. an immobilizable or immobilized avidin). For this purpose, avidin coated polystyrene beads are especially preferred. The biotinylated nucleic acid molecules and the avidin are incubated under conditions which permit the biotinylated molecules to bind to the avidin. The avidin-bound molecules are then removed from solution by centrifugation, sedimentation, affinity chromatography, or more preferably, by microcentrifuge filtration, etc., to produce a solution containing only the desired class 4 and s molecules.

As will be readily appreciated in view of the foregoing discussion, the present invention does not require the use of biotin and avidin. Such reagents are employed in the most preferred embodiment of the invention. Any pair of reagents capable of specific binding to one another can be used provided that one of the reagents can be linked to a nucleic acid molecule and that such a linked molecule will not be incapable of hybridizing with a complementary nucleic acid molecule. Examples of such alternative agents include modifying the nucleic acid molecules with an antigen (for example, digoxigenin) and purifying the undesired molecules from the mixture with an antigen specific antibody (such as antidigoxigenin antibody).

The direct effect of the affinity chromatography will be to enrich for those gene sequences which correspond to the sequence of the deletion. The nucleic acid obtained as the "flow-through" of the affinity chromatography, or as the solution resulting from the removal of the biotinylated nucleic acid bound-avidin beads, does not contain any biotinylated gene sequences. Such nucleic acid can be recovered and repeatedly cycled (using additional biotinylated, deletion-containing nucleic acid molecules. Such repeated cycles are capable of extensively enriching the concentration of a desired gene sequence.

In general, 3-5 such cycles are conducted in order to enrich a particular 5 kb yeast sequence to homogeneity. Since the yeast genome contains approximately 105 Sau3a fragments, and a 5 kb fragment would be expected to have approximately 20 Sau3a sites, the methods of the present invention, which are capable of isolating fragments containing each of these 20 sites, provides an enrichment of several thousand fold.

Once a sufficient degree of enrichment has been obtained, the enriched gene sequence can be introduced into a suitable cloning vector. The efficiency of cloning can be substantially enhanced if the enriched gene sequence is amplified prior to cloning. Although any method of amplification can be used, it is most preferable to amplify the gene sequences using the polymerase chain reaction described by Mull is in U.S. Patent No. 4,683,195, and by Saiki et al. (Science 239: 487-491 (1988)), which references are incorporated herein by reference. In a preferred embodiment, the fragments are "capped" by the ligation of adaptors that have sticky ends. Adapters having a Sau3A cleaved terminus comprise the preferred sticky end adapter. Alternatively, any linkers or adaptors may be used. If desired, such linkers or adaptors may be added to the termini of the molecules at any stage, even prior to the first hybridization. The ligated fragments can them be amplified by using a Taq polymerase and an excess of one adaptor strand which serves as a primer. Capping with adaptors and amplification is a general strategy for amplifying fragments of unknown sequence. Radioactive nucleotides are incorporated into the amplified DNA in a final round of polymerization. The labelled DNA is used to screen a genomic library made from wild-type DNA. The most intense signals should correspond to colonies that contain DNA coinciding with the sequences that are deleted in the mutant.

Once the DNA has been amplified, either by cloning or by the polymerase chain reaction, it can be used to screen any sort of library (i.e., cDNA, etc.) or blot (Northern or Southern). The amplified DNA can be cloned and used to screen blots or libraries. It is possible to use such a cloned amplified library with labeled amplified DNA in order to target clones that correspond to enriched sequences in the probe.

The concept of using subtractive hybridization was used to isolate mRNAs corresponding to DNA that had been deleted from a bacteriophage T4 deletion mutant (Bautz et al., Science 151:328-330 (1966). Several laboratories have recently published methods for enrichment of deleted DNA sequences from genomic DNA (Lamar et al., Cell 37:171-177 (1984); Welcher et al., Nucl. Acids Res. 14: 10027- 10044 (1986); Bjourson et al., Appl. Environ. Microbiol. 54:2852-2855 (1988)). The genomic subtraction procedure described herein makes it possible to achieve much greater enrichments than was possible using such methods. Enrichments obtained by single step competitive hybridization techniques such as the one developed by Lamar and Palmer (Lamar et al., Cell 37:171-177 (1984)) can be no greater than the ratio of deletion mutant to wild-type DNA. If a twenty fold excess of deletion mutant DNA is hybridized with wild-type DNA, one twentieth of all of the wild-type DNA strands will reassociate with another wild-type strand. This problem, which is a factor in any enrichment scheme that uses double stranded DNA, can be overcome either by using a great excess of deletion mutant DNA or by employing a iterative approach as we have done here. Subtraction protocols using single stranded cDNA and mRNA (Timberlake, Devel. Biol. 78:497-510 (1980)) do not require these measures since strands within the two nucleic acid populations cannot anneal to each other. Actual enrichments using the single step method have been less than 100 fold (Lamar et al., Cell 37:171-177 (1984); Kunkel et al., Proc. Natl. Acad. Sci. USA 82:4778-4782 (1985); Nussbaum et al., Proc. Natl. Acad. Sci. USA 84:6521-6525 (1987)). Two previous methods that employ multiple rounds of subtraction have resulted in the purification of sequences occurring in one strain of bacteria but not in another (Welcher et al., Nucl. Acids Res. 14:10027-10044 (1986); Bjourson et al., Appl. Environ. Microbiol. 54:2852-2855 (1988)). The enrichment factors required to achieve purification of the bacterial fragments were much smaller (about 200 fold smaller in reference 14) than in the experiment reported here. It is difficult to simultaneously obtain both impressive enrichments and useful amounts of DNA using techniques that lack an amplification step.

Thus, combining DNA subtraction with an amplification step allows attainment of large enrichment factors while minimizing the amount of DNA required. The methods of the present invention preferably accomplish such amplification by incorporating the polymerase chain reaction to amplify the DNA after the final round of subtraction. The polymerase chain reaction is described by Mullis in U. S. Patent No. 4,683,195, which reference is incorporated herein by reference.

Using the amplified DNA to probe a library greatly reduces screening time compared to screening Southern blots with individual clones. The signal generated by a particular sequence in the probe is proportional to its fractional representation in the probe. Thus, the fragments in the probe that are deleted in the mutant need not be purified in order to pinpoint the corresponding clones in a library. The desired fragments must only be more highly represented in the probe than any other particular fragment.

High enrichment factors depend on minimizing the accumulation of amplifiable contaminating species in the unbound DNA fractions. Two classes of fragments are expected to contaminate the unbound DNA: random and non-random. The random class is composed of reassociated wild-type strands that do not come from the deleted region, sequences that are single stranded because of insufficient reassociation times, and sequences that have annealed to mutant DNA that did not stick to the avidin coated beads. Possible non-random contaminants are fragments that can not hybridize to biotinylated DNA during subtraction (e.g. snap-back sequences or fragments with $T_m$'s below the hybridization temperature), those that correspond to a deletion at another locus, repetitive sequences that are much more numerous in the wild-type strain than in the deletion strain (in this case the biotinylated sequences may not be in sufficient excess), and DNA from organisms contaminating the wild-type strand.

## III. USES OF THE METHODS OF THE PRESENT INVENTION

The methods of the present invention can be used to generate a collection of restriction fragments from one strain that do not occur in another strain. These strains may differ in that one is distantly related to the other or in that one has been mutagenized with agents that cause high rates of deletion mutants. Oligonucleotides are synthesized that correspond in sequence to each strand of each such fragment. These oligonucleotides are pooled. DNA is made from a small sample of tissue and combined with the pooled oligonucleotides and then amplified using the polymerase chain reaction. If the tissue came from, or contained, the strain lacking the restriction fragments, no bands are apparent on an ethidium stained gel after amplification. However, if the tissue came from, or contained, the strain with the restriction fragments, one band appears corresponding to each fragment. The two strains can be crossed and the F1 progeny mated. The resulting F2 organisms are analyzed by amplification of DNA using the oligonucleotide primers followed by gel electrophoresis. The co-segregation of the fragments can be scored generating a linkage map of these fragments.

This approach simplifies the mapping of new genetic markers. The mutant, obtained in the strain containing the fragments, is crossed with the strain lacking the fragments and then the progeny (F1) mated with a sibling. The progeny of this cross (F2) are then scored for phenotype. DNA is made from a small sample of tissue and amplified using the pooled probes from the previous paragraph. The amplified products are scored for the presence and absence of the various fragments on a gel. In this way the phenotypic marker can be linked genetically to restriction fragment markers.

Similarly, phylogenetic trees can be constructed by isolating fragments that occur in one organism but that are absent (or have diverged so that they do not hybridize under the conditions used in the subtraction protocol) in a distantly related organism. Oligonucleotides are synthesized corresponding to such fragments. Tissue from related organisms could then be scored for relatedness to the two original organisms by simply amplifying a small amount of DNA using the oligonucleotides as primers. A phylogenetic tree can be constructed from the pattern of bands observed on a gel.

Similarly, a collection of human fragments can be obtained using this technique that are present in subsets of human populations. Oligonucleotides can be synthesized corresponding to each strand of each such fragment. DNA from a single cell forensic sample (such as a hair follicle) can be amplified using these pooled oligonucleotides as primers. The resulting band pattern is comparable to a fingerprint of the individual, and can be used in forensics in the same manner as a fingerprint.

The method can be used to isolate new pathogens (e.g., viruses). DNA from an infected individual can be treated as the wild type DNA in deletion cloning, DNA from uninfected tissue from the same individual or from another individual(s) (preferably both of the individual's uninfected parents) is treated as the deletion mutant DNA in deletion cloning.

The method can be used to isolate sex determining loci, disease resistance determining loci, disease causing loci, or drug resistance loci, if such loci contain unique restriction fragments present in one organism but not the other.

DNA that is absent in one tissue but present in another can also be isolated using the method. For example, a tumor might arise if both functional copies of a recessive oncogene are deleted. Using the method with tumor DNA and DNA from unaffected tissue would allow the recessive oncogene to be cloned.

The present invention also permits one to identify the nature of a bacterial, viral, yeast or fungal pathogen, and thereby provides an aid in diagnosing the etiology of infectious diseases. As described above, a collection of restriction fragments is made from one strain that do not occur in another strain. These strains may differ in that one is closely or distantly related to the other. Oligonucleotides are synthesized that correspond in sequence to each strand of each such fragment. These oligonucleotides are pooled. DNA from a small sample of tissue, or bodily fluid (blood, saliva, urine, stools, etc.) is obtained, combined with the pooled oligonucleotides and then amplified (preferably using the polymerase chain reaction). If the sample contained DNA from the strain lacking the restriction fragments, no bands are apparent on an ethidium stained gel after amplification. However, if the sample contained DNA from the strain with the restriction fragments, one band appears corresponding to each fragment. By comparing the obtained result with that generated by known strains, it is possible to identify the strain.

The application of genomic subtraction is subject to several constraints. Strains with homozygous or hemizygous deletions of the locus to be cloned must be available and viable. The deletion must cover at least one restriction fragment (for example, one Sau3A fragment) that is composed entirely of non-repetitive sequences. Ideally, the two strains should be isogeneic so that sequences covered by deletions occurring at irrelevant loci are not recovered.

Cloning DNA that corresponds to sequences missing in deletion mutants should be especially useful in systems in which mutants can be isolated but for which established cloning methods have not yet been developed. Because of the ease, efficiency and speed of genomic subtraction, this method may also prove valuable in organisms in which other cloning techniques such as transposon tagging or chromosome walking are commonly useful. For organisms with large genomes or those that exhibit polyteny, mutants can be screened cytologically for deletions. In the absence of cytological evidence, extensive genetic fine structure analysis is required to prove that a mutation is caused by a deletion. Rather than proving that a mutant is caused by a deficiency, genomic subtraction can be performed on a series of allelic mutants that have been induced by treatments that cause deletions at a high frequency. X-rays (Coté et al., Genetics 112:769-783 (1986); Kelley et al., Genetics 109:365-377 (1985)), diepoxybutane (Reardon et al., Genetics 115:323-331 (1987)), and chlorambucil (Russell et al., Proc. Natl. Sci. USA 86:3704-3708 (1989)) for example, can induce high proportions of deletion mutants. The methods of the present invention are designed in a way that facilitates processing multiple samples with a minimum of effort.

Thus, genomic subtraction can be used to isolate genes that cause human diseases in situations in which either patients with deletions can be found or cell lines that differ in the presence or absence of the locus can be identified. The method can also be used to isolate pathogen DNA from infected tissue. Virulence genes can be cloned from a pathogen if a related non-pathogenic strain exists. Clones obtained by applying the method to distantly related organisms can be used to probe intermediate species allowing the construction of phylogenetic trees. Similarly, DNA fragments that are present in one strain but absent in another can be used as RFLP markers. If appropriate oligonucleotides are synthesized, many markers can be evaluated simultaneously by amplification of genomic DNA. The technology we have developed can be applied to most experiments that require subtraction or positive selection of nucleic acids. The method may be extended to isolate DNA of higher plants and animals by accelerating the kinetics of the DNA reassociation reactions (Wieder et al., Biopolymers 20:1537-1547 (1981)).

Having now generally described the invention, the same will be more readily understood through reference to the following examples which are provided by way of illustration, and are not intended to be limiting of the present invention, unless specified.

## EXAMPLE 1

## PREPARATION OF DNA

In order to illustrate the generally applicable methods of the present invention, DNA was extracted from two strains of Saccharomyces cerevisiae: strain T1753 (alpha, ura3, trp1, his3, leu2, canr, cir°), and TD33.3 (alpha, ura3, trp1, his3, leu2, canr, cir° and Del(lys2)). The Del(lys2) mutation of strain TD33.3 contains a well characterized 5 kb deletion. The sequence corresponding to this deletion has previously been cloned and characterized. The cells were grown under conventional culturing conditions, harvested and treated to recover the genomic DNA.

DNA was extracted as described by Rogers et al. (Plant Molec. Biol. 5:69-76 (1985), which reference is herein incorporated by reference). DNA for genomic subtraction should be pure (i.e. free of DNA from biological contaminants, RNA, nucleotides, polysaccharides and proteins). It is important that genomic DNA concentrations be measured carefully. Genomic DNA concentrations are preferably determined by comparing band intensities after gel electrophoresis of the genomic DNA samples and several dilutions of bacteriophage 1 DNA of known concentration. Measurements of DNA concentration by absorbance spectroscopy are not generally sufficiently accurate for this application. For organisms that are rich in polysaccharides and/or glycoproteins, CTAB preps are useful (Murray et al., Nucleic Acids 8:4321-4325 (1980), which reference is incorporated herein by reference).

For the preparation of yeast DNA, pellets from 4 liters of saturated culture were resuspended in 240 ml 167 mg/ml sorbitol/0.2% (v/v) bME/0.1 mg/ml zymolase (Seikagaku; 105 units/mg) 100 mM EDTA. After incubation for 1 hr at 37°C the cells were spun for 20 min at 4°C in a Beckman JS4.2 rotor at 4200 rpm. The pellets were resuspended in 60 ml 50 mM glucose/25 mM Tris HCl pH 8/10 mM EDTA. To the resulting 100 ml suspension we added 25 ml of 3.5 M NaCl/0.1 M EDTA/0.5 M Tris HCl pH 8. DNA was prepared from this suspension according to the method of Murray and Thompson (Murray et al., Nuoleic Acids 8:4321-4325 (1980), Rogens, S. O. et al., Plant Molec. Biol. 5: 69-76 (1985)). The pellet from the final ethanol (EtOH) precipitation step was resuspended in 10 ml 10 mM Tris HCl pH 8/1 mM EDTA (TE). RNase A (Sigma) (Maniatis et al., Molecular Cloning: A Laboratory Manual (Cold Spring Harbor Laboratory, Cold Spring Harbor, New York) (1982)) was added at 100 mg/ml and the solution was incubated at 37°C for 1 hr. The solution was brought to 0.3 M NaOAc and extracted twice with phenol/chloroform (1:1). After adding 2 volumes of EtOH, DNA was spooled out of the solution, resuspended in 5 ml TE and then brought to 0.3 M Sodium Acetate (NaOAC). DNA was spooled out of EtOH solution twice more, washed in 100% EtOH and resuspended in TE. A portion of the genomic DNA isolated from strain TD33.3 was sheared by sonication to an average size of about 3000 bp using an Ultrasonics mode W-375 sonicator. The sheared DNA was concentrated to 0.5 ml by sec-butanol extraction, spun at 13,000 X g for 1 min to remove some insoluble material, denatured by boiling for 3 min, brought to 0.3 M NaOAC, precipitated by adding 2 volumes of EtOH, washed in 100% EtOH, dried, and resuspended at 1 mg/ml in water. Tris buffer was avoided at this stage since it may react with photobiotin.

A Sau3A adaptor was prepared by annealing the synthetic oligonucleotides GACACTCTC-GAGACATCACCGTCC and GATCGGACGGTGAT-GTCTCGAGAGTG. The latter oligonucleotides was phosphorylated at the 5' end using T4 polynucleotide kinase (New England Bioabs) (Maxam et al., Methods Enzymol 65:499-560 (1980)). An equal mass of each of the two strands were combined. The mixture was heated to 100°C for 2 min in a 200 ml water bath which was then allowed to cool to room temperature. At one end, the resulting adaptor has a Sau3A compatible sticky end with a 5' phosphate and at the other a Sau3A incompatible 5' overhang. The incompatible overhangs and single 5' phosphate insure that only one adaptor molecule is ligated onto each end of a Sau3A fragment.

The BglII fragment from the region deleted in strain TD33.3 was subcloned by ligating a BglII digest of YIp33.3 into pUC13 that had been cut with BamHI. The deleted fragment was excised from this plasmid by cutting with EcoRI and PstI (the BglII sites had been destroyed during subcloning). This fragment, referred to as the "5 kb BglII fragment" below, was gel purified on a 2% low-melting agarose gel (Ausubel et al., Current Protocols in Molecular Biology (Greene Publishing Associates and John Wiley & Sons, New York) (1987), which reference is incorporated herein by reference).

## EXAMPLE 2

## DNA MODIFICATION WITH PHOTOBIOTIN

Sheared DNA (1 μg/ml) in water, or in 10 mM tris HCl pH 8/1 mM EDTA (TE) that had been boiled for 2 minutes, and chilled in an ice bath, was mixed with an equal volume of photobiotin (1 μg/ml) (obtained from Vector Laboratories), or, more preferably, photobiotin acetate (1 μg/ml) (obtained from Clontech (Clonetech #5000-2 (5 mg in 2.5 ml water to a concentration of 2 μg/μl)) in a 2 ml polypropylene tube. The open tube was placed in a Styrofoam rack floating in an ice bath 10 cm underneath a GE Sunlamp (model RSM/H, equipped with a 275 W bulb). After approximately fifteen minutes the lamp was turned off and the reaction was brought to 100 mM tris HCl pH 9 by adding a 1 M stock solution. Unreacted photobiotin was removed by extracting three, and preferably four, times with water saturated n-butanol. After ethanol precipitation the modified DNA was resuspended at 2 mg/ml in TE (10 mM Tris HCl, 1 mM EDTA, pH 8), or more preferably, EE (EE is 10 mM (Na)Epps, pH 8.25; 1 mM EDTA, pH 8; adjusted to a final pH of 8.0. EE is made as 10 X EE using 1 M (Na)Epps, pH 8.25, and 0.5 M EDTA, pH 8). EPPS (N-(2-hydroxyethyl) piperazine-N'-3-propanesulonic acid) is a close chemical relative of HEPES. Its $pK_a$ is 8.0 (at 20°C) and the $pK_a$ changes only slightly with temperature (-0.007 units/°C). The pH of 1 X EE at 100°C should be about 7.4. Keeping the pH neutral is important for this procedure in order to avoid depurination of DNA. This occurs rapidly at acid pH and high temperatures. Tris is inappropriate since the $pK_a$ drops rapidly with increased temperature.

It is important to maintain a neutral pH during extensive high temperature incubations in order to minimize depurination of DNA (Lindahl et al., Biochemistry 11:310-318 (1972)). EPPS was used because its $pK_a$ (8.0 at 20°C) changes slowly with temperature (-0.007 units/°C. A 1 M stock solution of EPPS pH 8.25 was used to make all EPPS containing buffers.

## EXAMPLE 3

## DNA REASSOCIATIONS

Sau3A digested T1753 DNA (0.5 $\mu$g), biotinylated DNA from strain TD33.3 (10 $\mu$g), an end-labelled 84 base synthetic oligonucleotide (70,000 cpm), and 40 $\mu$g yeast tRNA (Sigma) were denatured in 2 X EE by boiling for 1 min. Lyophilized yeast tRNA is resuspended in 0.3 M NaOAc. It is preferably extracted twice with Phenol/chloroform (1:1) and EtOH precipitated, and resuspend such that the concentration is 20 $\mu$g/$\mu$l, prior to use.

The 84-mer, which does not hybridize with yeast DNA, had been end labelled using T4 polynucleotide kinase (New England Biolabs) and $\alpha$-$^{32}$P-ATP (New England Nuclear).

The oligonucleotides molecule was included as a marker for the fractions containing DNA that did not bind to avidin and was used to estimate overall yield. Any radioactive DNA, so long as it is not homologous to the modified DNA, can be used to monitor the efficiency of the reactions and to pinpoint the fractions containing DNA during affinity chromatography. The ideal tracer should be long enough to resemble a Sau3a fragment; single stranded (so as to indicate the fate of the single stranded molecules that stick more readily to the sides of tubes, etc.; and must not hybridize to the genome at 1 M NaCl, 65° C.). Labelled DNA (100,000 cpm) and tRNA (20 $\mu$g) need only be added to the first reassociation reaction.

The mixture was brought to 1 M NaCl/5 mM EDTA/ 100 mM Hepes pH 7.5 (1 x SHE) by adding 4 x SHE. Final DNA concentrations are between 1 and 2 mg/ml. The mixture was allowed to reassociate for 21-44 hr at 65° C. Reactions reached $C_ot$ values greater than or equal to twenty times the $C_ot_{1/2}$ value for yeast DNA under these conditions.

Alternatively, and more preferably, the mixture was lyophilized, resuspended in 4 $\mu$l 2.5 X EE, and then mixed with 1 $\mu$l 5 M NaCl. Hybridizations were carried out in a 65° C air incubator in 0.5 ml centrifuge tubes to maintain constant sample volumes. After 17 hr at 65° C, 95 $\mu$l of EEN (EEN is 0.5 M NaCl; 10 mM (Na)Epps, pH 8.25; 1 mM EDTA, pH 8; adjusted to a final pH of 8.0) was quickly and thoroughly mixed with the sample which was then combined with 100 $\mu$l of a 5% suspension of avidin coated polystyrene beads (Fluoricon™ Avidin polystyrene Assay Particles, Baxter Healthcare Corporation, Pandex division, #31-040-1 (0.7-0.9 $\mu$ diameter) (Syvänen et al., Nucl. Acids Res. 16:11327-11338 (1988)) that had been washed in EEN. The sample was incubated at room temperature for 30 min, added to the cup of a microcentrifuge tube filter unit (Millipore, #UFC3, OGV 00), and spun at 13,000 X g for 15 sec. The beads, which were retained by the filter, were washed once with 200 $\mu$l EEN. The unbound nucleic acid in the filtrate was precipitated at -70° C following the addition of two volumes of ethanol. The resulting pellet was washed with ethanol, dried, and resuspended in 5 $\mu$l EE. 0.5 $\mu$l for subsequent analysis. The sample was combined with 10 $\mu$g biotinylated TD33.3 DNA, 20 $\mu$g yeast tRNA, and brought to 2 X EE. Four more cycles of denaturation, reassociation, and avidin selection were performed as above. Aliquots (1/10 of each unbound fraction) were saved after each cycle. tRNA (20 $\mu$g) was added as needed to maintain a visible pellet after centrifugation. The recovery of DNA not capable of hybridization ranged from 80-90% per round, as judged by the loss of the labelled oligonucleotide. After 5 rounds of subtraction, the recovery of the labelled oligonucleotide was 44% of the theoretical yield.

If desired, the above protocol can be modified to permit recovery of the bound DNA. The beads are washed with EEN, the bound DNA eluted with 100 mM NaOH, and the filtrate is neutralized.

Fragments that have $T_m$'s near or below 65° C in 1 M NaCl cannot hybridize efficiently under the hybridization conditions described above, and thus will be found in the unbound fraction after each round of subtraction. To insure that such fragments were completely denatured so that they could not be capped by adaptors and thus could not be amplified, a high temperature incubation step is preferably performed prior to the addition of the adaptors.

For this step, approximately 20% of the DNA that was saved after cycles 1-4 and 10% of the unbound fraction from cycle 5 was resuspended in 50 $\mu$l 1 M NaCl/EE/ 400 $\mu$g/ml tRNA. After incubation at 80° C for 30 min, EE (200 $\mu$l) was added, the samples were precipitated and washed with EtOH, and DNA was resuspended in 5 $\mu$l EE. Half of the DNA from each sample was ligated to Sau3A adaptors (50 ng) using 6 units of T4 DNA ligase (Pharmacia) in 10 $\mu$l at 15° C for 7 hours. In addition, Sau3A digested T1753 DNA and the cloned, Sau3A cut, gel purified, BglII fragment were ligated to adaptors.

## EXAMPLE 4

## BIOTIN-CELLULOSE CHROMATOGRAPHY

In one embodiment of the present invention, the preparation of biotinylated and non-biotinylated nucleic acid molecules are incubated in the presence of unbound avidin, in order to remove biotinylated molecules from the sample.

To accomplish such a procedure, avidin (10 $\mu$l of 1 mg/ml avidin per $\mu$g modified DNA) was added to the reassociation reaction and thoroughly mixed at room temperature. The reaction was brought to 1 x SHE by adding 4 x SHE, and the mixture was incubated at room temperature for 30 min. Biotin cellulose was prepared by mixing a 50% slurry (30 $\mu$l slurry/$\mu$g modified DNA) with 4 x SHE (333 $\mu$l/ml slurry) and yeast tRNA (100 $\mu$g/ml slurry). The matrix was then washed twice by mixing with 50 ml 1 x SHE and spinning for 1 min in a clinical centrifuge. The avidin:DNA mixture was added to the washed biotin-cellulose pellet, shaken at room temperature for 30 min and poured into a plastic column or disposable pipette tip plugged with siliconized glass wool. Alternatively, the DNA can be bound by passing the mixture over a biotin-cellulose column directly. The column was washed with 1 x SHE and fractions containing radioactivity are collected. These fractions are extracted with 50% phenol/50% chloroform, concentrated two fold by sec-butanol extraction, if desired, and then combined with two volumes of ethanol. After incubation for 5 min in a dry ice:ethanol bath and centrifugation for 5 min at 15,000 g, the nucleic acid was resuspended in 0.5 ml 0.3 M sodium acetate and allowed to precipitate as before. The dried pellet was resuspended in TE and may be combined with fresh biotin modified DNA or amplified.

## EXAMPLE 5

## DNA AMPLIFICATION AND COLONY HYBRIDIZATION

The DNA samples of Example 4 were subjected to 4 rounds of subtractive hybridizations and affinity chromatography. After each round, samples were amplified using the polymerase chain reaction or other means. Amplified DNA was labeled with $^{32}$P and used to probe replicas of a yeast genomic library. Approximately $10^5$ yeast genomic colonies were blotted to each filter. After 4 rounds of enrichment and amplification, additional rounds of enrichment and amplification did not change the number of colonies capable of hybridizing to the amplified DNA, or the specific colonies which hybridized to the labeled DNA. This result indicates that the method was successful in enriching for the desired gene sequences (i.e. such that the same amount of DNA contained fewer species in addition to the desired gene sequence, and thus were capable of hybridizing to fewer colonies in the genomic library.

In a subsequent experiment, the colonies which were found to be capable of hybridizing to the enriched and amplified labeled DNA was tested for its ability to hybridize to the previously cloned sequence which was known to correspond to the Del(lys2) deletion. All of the colonies which were capable of hybridizing to the DNA present after the fourth round of enrichment and amplification were also capable of hybridizing to sequences known to correspond to the Del(lys2) deletion. This results shows that the method actually resulted in the enrichment of the desired gene sequence.

## EXAMPLE 6

## PREFERRED METHOD FOR SUBSTRACTION

To demonstrate this embodiment of the invention, the above-described yeast strains TD33.3 (missing a 5 kb BglII fragment covering the lys2 gene), and T1753 were again used to clone the lys2 region.

For this embodiment of the invention, DNA was purified and biotinylated as described above. The nucleic acid having an insertion, or containing DNA corresponding to the deletion whose cloning was desired is incubated in the presence of Sau3A (a 10 fold overdigestion is acceptable). Restricted DNA was resuspended at 0.1 $\mu$g/$\mu$l in EE. Biotinylated DNA from a source carrying the deletion and non-biotinylated from a source having DNA which corresponds to the deletion were denatured, mixed, and permitted to hybridize with complementary strands as described above.

Rather than using free avidin in solution as a means for removing the unwanted biotinylated DNA, such DNA was removed from the sample by permitting it to bind to polystyrene beads which had been previously coated with avidin. Commercially available avidin-coated beads were treated to remove azide or free avidin, which may be associated with such beads. To accomplish such removal, 2 ml 5% (w/v) Avidin beads were centrifuged for 1 minute in micro-centrifuge. The supernatant was recovered and discarded. The pellet (containing the avidin beads) was resuspended in 1 ml EE buffer. The suspension was centrifuged and washed two additional times, each using 1 ml of EE buffer. The final pellet was resuspended with EEN to 1.8 ml or so (in consideration of the fraction of the AP beads which were estimated to have been lost in the washings).

DNA was sonicated in ~3 ml EE in 15 ml disposable tubes. Typically one would shear a few hundred $\mu$g of DNA carrying the deletion, preferably for 15 seconds using an Ultrasonics model W-375 sonicator with a microtip in a continuous (i.e. not pulse) mode with an output setting to 5. Such treatment resulted in a DNA preparation whose DNA had an average length ~3 Kb. The average length of the DNA is not critical. However, the sheared DNA should be sufficiently long that the chance of a fragment not reacting with photobiotin is negligible. Since about 1 in 100 nucleotides will be biotinylated, the fragments should be quite a bit larger than 100 bp.

The DNA was concentrated to 0.5 ml using sec-Butanol ("sec-BuOH"). Approximately 2.5 ml of sec-BuOH was added per ml of water. The mixture was vortexed for approximately 10 sec, and centrifuged in a clinical centrifuge for approximately 30 sec. The upper phase (sec-BuOH) was removed and discarded. Optionally, one may re-extract with sec-BuOH, using a volume of sec-BuOH equal to the current aqueous phase volume. The aqueous phase was boiled for 3 minutes, and 50 $\mu$g 3 M NaOAc was added, and the sample was mixed. When using yeast DNA, a white precipitate may form when the NaOAc is added after the sec-BuOH concentration. This material is believed to be a polysaccharide, and may be removed by centrifugation.

Approximately 1 ml EtOH was then added to the sample, in order to precipitate the DNA. EtOH precipitation was accomplished by incubating the sample for 5 minutes in a dry ice/EtOH bath. The mixture was thereafter centrifuged for 5 minutes in a microcentrifuge, washed in 100% EtOH, and dried.

DNA was resuspended in water at 1 $\mu$g/$\mu$l. Since Tris may react with the photobiotin, it is preferred to resuspend in water rather than in a Tris buffer (such as TE).

DNA was biotinylated as follows: 1 volume of 2 $\mu$g/$\mu$l photobiotin (in water) was mixed with 1 volume of 1 $\mu$g/$\mu$l sheared DNA (in water) in a microcentrifuge tube. An aliquot (100 $\mu$l) of the DNA/photobiotin mixture was pipetted into the bottom of a 2 ml microcentrifuge tubes (with wide angle conical bottoms). The tube (cap open) was floated in an ice bath 10 cm below a sun lamp as described above. After photobiotinylation has occurred, 0.2 volumes of 1 M Tris pH 9 were added. This corresponded to 10% of the current volume.

The sample was extracted 4 times with water-saturated n-BuOH, and the bottom (aqueous) phase was saved each time. The volume of sample was brought to 0.45 ml, and 50 $\mu$l 3 M NaOAc was added to the microcentrifuge tube. The sample was ethanol precipitated as described above, and the final sample was resuspended in 0.4 volumes of 2.5 X EE (ie.e at 2.5 $\mu$g/$\mu$l). The final sample was then ready for use in subtractive hybridization.

## EXAMPLE 7

### SUBTRACTIVE HYBRIDIZATION

For the first subtractive hybridization, 5 $\mu$l of a 0.1 $\mu$g/$\mu$l Sau3A digested " + " DNA (containing DNA corresponding to the deletion sought to be cloned), 4 $\mu$l of 2.5 $\mu$g/$\mu$l sheared photobiotinylated "-" DNA (containing DNA carrying the deletion), 3 $\mu$l 10 X EE, 1 $\mu$l labeled tracer DNA (~$10^5$ cpm of the previously discussed synthetic 84-mer), and 2 $\mu$l $\mu$g/$\mu$l yeast tRNA. The mixture was boiled for 1 min, lyophilized, and resuspended in 4 $\mu$l water. 1 $\mu$l 5 M NaCl was added, and the sample was mixed and then centrifuged. The sample was then incubated in a 65°C air incubator to $C_0t \geq 20 \times C_0t_{1/2}$.

The hybridized DNA was incubated with the previously prepared avidin coated beads. The hybridization reaction was brought to a volume of 100 $\mu$l with EEN with vortexing. 100 $\mu$l of washed 5% Avidin coated polystyrene beads in EEN were added to the 100 $\mu$l hybridization mixture. The mixture was then incubated for 30 minutes at room temperature.

Unbound DNA was collected by pipetting the sample/bead mixture into the cup of a microcentrifuge filter unit (Millipore, #UFC3 OGV 00 is a suitable microcentrifuge tube with a removable cup containing a 0.2 $\mu$m PVDF filter). The unit was then centrifuged for 15 seconds in a microcentrifuge. The tube used for the hybridization and the pipette tip was then washed with 200 $\mu$l EEN and this wash solution was added to the cup of the filter unit. The unit was then centrifuged for an additional 15 sec (the volume of the filtrate in the filter unit tube was ~400 $\mu$l. Approximately 1 ml EtOH was added to the filtrate, and the sample was EtOH precipitated as described above.

In order to perform subsequent subtractive hybridizations, the pellet was resuspended in the presence of 4 $\mu$l 2.5 $\mu$g/$\mu$l sheared photobiotinylated "-" DNA in 2.5 X EE. If one desires to save a sample for amplification, then the pellet should be resuspended in 4.4 $\mu$l of DNA and 0.4 $\mu$l saved by diluting it into 5 $\mu$l of TE (prior to boiling). If one is concerned about having biotinylated genomic DNA in the sample, then the pellet should be resuspended in TE, an aliquot (e.g. 1/10th) should be saved, and the bulk of the sample lyophilized, and resuspended in 4 $\mu$l of 2.5 $\mu$g/$\mu$l biotinylated DNA.

Resuspended sample which was to be used for additional subtractive hybridizations was then boiled for 1 minute, after which, 1 $\mu$l 5 M NaCl was added. The sample was then mixed and centrifuged, and the amount of radioactive tracer determined by Cerenkov counting. The sample was then incubated in a 65°C air incubator, preferably under paraffin oil to minimize any evaporation of solvent, to $C_0t \geqq 20 \times C_{t1/2}$. Unbound DNA was collected as described above.

Fragments that have $T_m$'s near or below 65°C in 1 M NaCl cannot hybridize efficiently under the hybridization conditions used in these experiments and thus will be found in the unbound fraction after each round of subtraction. To insure that such fragments were completely denatured so that they could not be capped by adaptors and thus could not be amplified, the above-discussed high temperature incubation step is preferably conducted prior to the addition of the adaptors.

Thus, DNA from wild-type strain T1753 was cut with Sau3A. DNA from strain TD33.3 was sheared by sonication to average size of 3000 bp. Starting with 0.5 $\mu$g of wild-type DNA and using 10 $\mu$g of biotinylated deletion mutant DNA in each round, 5 cycles of subtraction were performed on the sample prepared in accordance with Example 6. After each cycle, an aliquot of the unbound DNA onto which Sau3A adaptors were ligated was saved. A fraction of each aliquot was amplified using the polymerase chain reaction.

For such amplification, a fraction (e.g. 1/10th) of the unbound DNA was added to a 500 $\mu$l tube containing: 44 $\mu$l water; 10 $\mu$l 5 M NaCl; 5 $\mu$l 10 X EE; and 1 $\mu$l 20 $\mu$g/$\mu$l yeast tRNA. The mixture was incubated at 80°C for 30 min in a PCR (Polymerase Chain Reaction) machine or water bath. At the conclusion of the incubation, 200 $\mu$l EE was added to the sample, and the DNA was EtOH precipitated in 500 $\mu$l EtOH as described above (-70°C, 5 min; centrifuge, 5 min; wash with 100% EtOH, dry. After such precipitation, the DNA was resuspended in 5 $\mu$l EE.

To amplify the DNA, adapters of the termini were added. It is desirable to ligate adaptors onto a Sau3A digest of vector DNA. This will serve as a positive control in the amplification reaction. It may also be useful to ligate adaptors onto the original Sau3A digested " + " DNA.

To add such adapters, 2.5 $\mu$l "melted" unbound DNA was mixed with 2 $\mu$l 25 ng/$\mu$l Sau3A adaptor, 1 $\mu$l 10 X ligation buffer, 4 $\mu$l TE, and 0.5 $\mu$l T$_4$ DNA ligase. The reaction mixture was incubated at 15°C for $\geqq$ 2 hr.

## EXAMPLE 8

## DNA AMPLIFICATION AND COLONY HYBRIDIZATION

For DNA amplification, DNA capped with adaptors (1/10 of the ligation reaction) was amplified in a thermal cycler (Ericomp) with the GeneAmp™ Kit (Perkin-Elmer Cetus Instruments) using 0.5 mg of phosphorylated adaptor strand GACACTCTCGAGACATCACCGTCC as a primer. The primer was phosphorylated to facilitate the subsequent cloning of the amplified DNA. Each of the 50 cycles included 3 segments: 30 sec at 93°C, 30 sec at 55°C, and 3 min at 72°C. 0.5 $\mu$g of phosphorylated adaptor strand is used to prime the polymerase reaction. It is important to use the correct strand (i.e. the strand whose 3′ end is part of the adaptor's Sau3A site). The strand is prefereably phosphorylated to facilitate cloning of the amplified DNA. The following controls are useful: ~ 10 fg " + " DNA cut with Sau3A capped with adaptors; ~ 10 fg vector DNA cut with Sau3A capped with adaptors; and a Primer only control (no template).

Amplified DNA (1/10 of the sample) was denatured in the presence of adaptor strand GACACTCTCGAGACATCACCGTCC (0.2 $\mu$g) and labelled using [$\alpha$-$^{32}$P]dCTP and Klenow fragment of DNA polymerase I).

Free nucleotides were removed from the amplified DNA by spinning samples through columns (Maniatis et al., Molecular Cloning: A Laboratory Manual (Cold Spring Harbor Laboratory, Cold Spring Harbor, New York) (1982)) composed of extensively washed Sephadex-G25 (Pharmacia).

An electrophoretic analysis of the amplified DNA derived from cycles 1-3 was performed using a 2% agarose gel containing ethidium bromide (EtBr) (0.25 $\mu$g/$\mu$l). The products from rounds 4 and 5 looked identical to those from round 3. The products obtained upon amplification of 10 fg of the Sau3A cut cloned BglII fragment was also detected. The pattern of bands obtained after 3 cycles looks nearly identical to the pattern of the amplified Sau3A cut, cloned BglII fragment that is deleted in strain TD33.3. This indicated that a high degree of enrichment was achieved for the fragments corresponding to the deletion after 3 rounds of subtraction. Comparing the amplified Sau3A digest of the cloned deleted fragment to the unamplified digested fragment, it was apparent that several large bands were not amplified efficiently. Similarly, comparison of unamplified and amplified Sau3A cut T1753 DNA indicated that fragments larger than about 700 bp were not efficiently amplified. The fragments in the unampilfied samples were not capped with adaptors and thus migrated faster than corresponding fragments in the other lanes.

The amplified DNA from rounds 2 and 3 was labelled and used to probe replica filters made from a plate containing about $10^5$ yeast genomic plasmid clones. For such colony hybridization, amplified DNA (1/10 of the sample) wad denatured in the presence of adaptor strand GACACTCTC-GAGACATCACCGTCC (0.2 $\mu$g) and labelled using [$\alpha$-$^{32}$P]dCTP and Klenow fragment of DNA polymerase I (Ausubel et al., Current Protocols in Molecular Biology (Greene Publishing Associates and John Wiley & Sons, New York) (1987)). Plasmid Ylp33.3 containing the cloned yeast lys2 gene was digested with BglII and XhoI. The two fragments comprising the BglII fragment that is deleted in strain TD33.3 were gel purified and labelled using random hexamers as primers (Feinberg et al., Anal. Biochem. 137:266-267 (1984)). Replica filters containing about 1 X $10^5$ yeast genomic clones were hybridized to the labelled DNA (Hanahan et al., Gene 10:63-67 (1980); Grunstein et al., Proc. Nat'l. Acad. Sci. USA 72:3961-3965 (1975)).

The cloned 5 kb BglII fragment which is missing in strain TD33.3 was labelled and hybridized to a replica filter made from the same plate. Genomic clones containing sequences that are deleted in strain TD33.3 were observed by autoradiogram of 3 replica filters, as spots which hybridized to the labelled 5 kb BglII deleted fragment. After hybridization with the probe derived from the third cycle, the prominent signals corresponded to spots generated by the labelled 5 kb BglII fragment. Thus, 3 rounds of genomic subtraction provided sufficient enrichment to identify clones containing sequences that are absent in the deletion mutant. Most of the colonies that hybridize to the probe from round 2 do not contain deleted sequences; however, several of the darker signals are superimposable on the spots on the filter that was probed with the cloned deleted sequences indicating that some enrichment for deleted sequences has occurred after 2 cycles of subtraction.

A small fraction of the clones that are pinpointed by the cloned deletion probe did not appear as spots on the round 3 filter. Analysis of duplicate filters probed with the round 3 probe indicate that the spots are missing due to unfaithful replica plating. Also, in an independent genomic subtraction experiment all of the clones that hybridized to the cloned deletion probe were also pinpointed by the experimental probe.

Analysis of cloned amplified unbound DNA indicated average enrichments of approximately 10 fold during each of the first three rounds. About 95% of the clones derived from the fourth round hybridized to the deleted sequences.

Results similar to those reported above were obtained in two additional independent genomic subtraction experiments. In one experiment, 5 rounds of subtraction were required to obtain results comparable to that described above. In the other experiment (Examples 4 and 5), DNA was incubated with free avidin and bound the samples to a biotin-cellulose matrix instead of using avidin coated beads (Kasher et al., Mol. Cell. Biol. 6:3117-3127 (1986)). Enrichments comparable to those reported here were obtained after 4 cycles in the latter experiment.

Attempts to perform genomic subtraction when adaptors were added at the beginning of the experiment (i.e. after Sau3A digestion of the wild-type DNA) have not resulted in enrichments equal to those shown here. This may be due to the amplification of significant amounts of DNA that remains unbound during the affinity steps for reasons other than that they correspond to a chromosomal deletion.

In summary, using genomic subtraction we efficiently isolated sequences that are absent in a deletion that spans 1/4000$^{th}$ of the yeast genome. Cloning sequences that correspond to deletions that represent smaller fractions of a genome should be possible simply by performing more rounds of subtraction.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth as follows in the scope of the appended claims.

## Claims

1. A method for enriching for a desired gene sequence absent in one population of nucleic acid molecules but present in another which comprises:

a) mixing a first population of denatured nucleic acid molecules suspected of possessing said desired gene sequence with an excess amount of a second population of denatured nucleic acid molecules suspected of lacking said desired gene sequence, wherein said second population of molecules has been labelled with a label, and wherein said first population of molecules lacks said label;

b) permitting the mixed molecules of said first and second populations of molecules to hybridize and thereby form double-stranded nucleic acid molecules; and

c) isolating any double-stranded molecules of said mixture which are composed solely of renatured molecules of said first population.

2. The method of claim 1 wherein said label of said labelled second population of molecules is a biotin label, or an antigen label.

3. The method of claim 1 wherein said step (c) comprises the sub-steps:

i. incubating said mixed first and second populations of molecules with an agent capable of binding to said label of said second population of molecules;

ii. permitting said agent to bind to said label of said second population of molecules; and

iii. separating any of said second population of molecules from said mixture by selectively separating said bound agent, along with its bound, labelled, molecule of said second population of molecules, from said mixture.

4. The method of claim 3 wherein said label of said labelled second population of molecules is a biotin label.

5. The method of claim 3 wherein said agent is an antibody.

6. The method of claim 3 wherein said agent is avidin.

7. The method of claim 6 wherein said avidin is in solution.

8. The method of claim 7 wherein said avidin in solution, after binding to said labelled molecule of said second population of molecules, is removed from solution along with said bound, labelled, molecule by affinity separation with biotin, or by immunoaffinity separation with an anti-avidin antibody.

9. The method of claim 6 wherein said avidin is bound.

10. The method of claim 9 wherein said avidin is bound to a bead or a resin.

GENOMIC SUBTRACTION

RESTRICTED WILD TYPE DNA

BIOTINYLATED, SHEARED, DELETION
MUTANT DNA (IN EXCESS)

DENATURE, REASSOCIATE

AVIDIN COATED
BEADS

BOUND DNA

UNBOUND
DNA

ADAPTOR LIGATION

PCR
AMPLIFICATION

ADAPTOR STRAND
PRIMER

RADIOACTIVE
LABELLING

\*          \*

FIG. 1